# FASCICULE DE BREVET EUROPEEN

(11) **EP 2 939 007 B1**
(45) Date de publication et mention de la délivrance du brevet: **11.07.2018**
(21) Numéro de dépôt: 13820813.7
(22) Date de dépôt: 17.12.2013
(51) Int. Cl.: G01N 22/00, G01N 21/17, G01N 27/22, G01N 33/00

(54) **PROCEDE ET DISPOSITIF POUR CARACTERISER UN MILIEU FLUIDE A L'AIDE D'UN SUBSTRAT SEMI-CONDUCTEUR**
VERFAHREN UND VORRICHTUNG ZUR CHARAKTERISIERUNG EINES FLUIDMEDIUMS MIT EINEM HALBLEITENDEN SUBSTRAT
METHOD AND DEVICE FOR CHARACTERISING A FLUID MEDIUM USING A SEMI-CONDUCTIVE SUBSTRATE

(30) Priorité: 27.12.2012 FR 1262879
(43) Date de publication de la demande: 04.11.2015
(73) Titulaire: Centre National de la Recherche Scientifique (C.N.R.S.), 75794 Paris Cedex 16 (FR); Ecole Centrale De Lyon, 69134 Ecully Cedex (FR); Institut National des Sciences Appliquées de Lyon, 69621 Villeurbanne Cedex (FR); Université Claude Bernard Lyon I, 69622 Villeurbanne Cedex (FR)
(72) Inventeur: LYTVYNENKO, Sergeii, Kiev 01601 (UA); ALEKSEYEV, Sergei Alexandrovich, 69100 VILLEURBANNE (FR); LYSENKO, Volodymyr, F-69621 Villeurbanne (FR); SKRYSHEVSKYY, Valeriy, Kiev 01601 (UA)
(74) Mandataire: Be IP
(86) Numéro de dépôt international: PCT/FR2013/053134
(87) Numéro de publication internationale: WO 2014/102484

(56) Documents cités:
- EP-A1- 2 037 288
- WO-A1-94/14188
- WAGNER T ET AL: "A high-density multi-point LAPS set-up using a VCSEL array and FPGA control", PROCEDIA CHEMISTRY, ELSEVIER, AMSTERDAM, NL, vol. 1, no. 1, 1 septembre 2009 (2009-09-01), pages 1483-1486, XP026799837, ISSN: 1876-6196 [extrait le 2009-09-01] cité dans la demande

## Description

La présente invention concerne le domaine technique de la reconnaissance ou de la caractérisation d'un milieu fluide au sens général, à l'aide d'un substrat semi-conducteur.

L'identification de milieux fluides et/ou de produits ou substances contenus dans des milieux fluides trouvent de nombreuses applications comme par exemple dans la détection de produits ou substances dans un milieu fluide, le contrôle de qualité ou le contrôle de conformité, etc.

Dans l'état de la technique, il est connu par la publication « A high-density multi-point LAPS set∼up using a VCSEL array and FPGA control », Sensors and Actuators B154 (2011) 124-128, un capteur désigné par l'acronyme L.A.P.S. (pour Ught-Adressabie Potentiometric Sensor) dont la structure comporte un transducteur potentiométrique en contact avec le liquide à analyser et séparé d'un élément semi-conducteur par un isolant. Une source de lumière modulée est utilisée pour créer une excitation photonique localisée qui pénètre directement à l'intérieur de l'élément semi-conducteur pour générer des paires d'électrons-trous à l'intérieur dudit élément. L'application d'une tension de polarisation aux bornes du capteur conduit à la génération d'un photocourant. La valeur du photocourant dépend en particulier de la tension de polarisation, de l'excitation photonique et du potentiel à l'interface liquide/isolant.

Il ressort du principe même de ce capteur que le signal de photocourant est de nature photocapacitive en raison de l'interface isolant/semi-conducteur. Il s'ensuit que la sensibilité d'un tel capteur est faible et que le signal détecté est de faible puissance. Par ailleurs, il apparait que les zones non éclairées influencent le signal crée par les zones localisées éclairées.

Dans l'état de la technique, il est également connu par la publication « Chemical Sensors for Electronic Nose Systems », Microchim. Acta 1.49, 1-17 (2005), un système de nez électronique mettant en oeuvre un capteur chimique pour permettre l'analyse de composés organiques volatiles. Un tel système comporte un transistor à effet de champ MOSFET correspondant à l'acronyme (Metal Oxide Semiconductor Field Effect Transistor). La grille du transistor est en contact avec le gaz à analyser qui est séparé de la jonction drain-source par un isolant. Les charges sur l'isolant influencent par un effet de champ, le courant drain-source et par suite le photocourant recueilli aux bornes du transistor permettant ainsi de caractériser le gaz à analyser.

Il ressort du principe même de ce nez électronique que le signal de courant entre le drain et la source est modulé par effet de champ via l'interface isolant/fluide. Un inconvénient de ce nez électronique concerne la nécessité de disposer d'une grille spécifique pour assurer une caractérisation sélective des gaz. A cet égard, pour être sensible aux composés neutres, par exemple, des catalyseurs spécifiques doivent être utilisées afin de décomposer les molécules neutres en éléments chargés. Un autre inconvénient de ce nez électronique (du point de vue de sa réalisation technologique) concerne la nécessité d'isoler électriquement le drain et la source de chaque transistor du fluide à analyser. La résolution spatiale de ce dispositif est définie et limitée par la taille de chaque transistor.

Dans le domaine technique visant à déterminer la pureté de matériaux semi-conducteurs en examinant la durée de vie des porteurs minoritaires, il est connu d'utiliser une technique micro-ondes de mesure associée à une source laser puisée. Par exemple, le document WO 94/14188 propose, pour mesurer la durée de vie de porteurs minoritaires d'un substrat semi-conducteur, d'entourer le substrat semi-conducteur à l'aide d'une solution de passivation, et de le soumettre à une source micro-ondes et à une source lumineuse pulsée afin de créer des charges électriques photogénérées. L'énergie micro-ondes réfléchie est détectée, permettant de mesurer la durée de vie des charges électriques photogénérées. A partir de cette mesure, il est ainsi possible de détecter les défauts du substrat semi-conducteur,

La présente invention vise à remédier aux inconvénients de l'art antérieur en proposant une nouvelle méthode pour caractériser à l'aide d'un substrat semi-conducteur, un milieu fluide c'est-à-dire un liquide et/ou un gaz et/ou des produits ou substances contenus dans des milieux fluides, de nature électriquement neutre ou non, cette méthode de caractérisation présentant une sensibilité élevée et une résolution spatiale adaptable facilement.

Pour atteindre un tel objectif, l'objet de l'invention propose un procédé pour caractériser un milieu fluide à l'aide d'un substrat semi-conducteur.

Selon l'invention, le procédé comporte les étapes suivantes :
- réaliser une surface de réception pour le milieu fluide sur au moins une face du substrat semi-conducteur,
- mettre le milieu fluide à caractériser en contact avec la surface de réception, afin de réaliser une interface substrat/milieu fluide,
- éclairer à travers le milieu fluide, au moins une zone de l'Interface à l'aide d'un faisceau de lumière puisé pour créer des charges électriques photogénérées,
- en utilisant un réflectomètre à micro-ondes, mesurer la durée de vie des charges électriques photogénérées dont la valeur dépend de la vitesse de recombinaison à l'interface substrat/milieu fluide,
- créer une matrice des valeurs des durées de vie mesurées, des charges électriques photogénérées,
- déterminer une signature électronique caractéristique du milieu fluide à partir de la matrice des durées de vie créée,
- analyser la signature électronique afin de reconnaître le milieu fluide et/ou l'un ou plusieurs de ses composants élémentaires.

De plus, le procédé selon l'invention peut présenter en outre en combinaison au moins l'une et/ou l'autre des caractéristiques additionnelles suivantes ;
- éclairer successivement dans le temps, plusieurs zones de l'interface et à déterminer Ses durées de vie correspondantes des charges électriques photogénérées, pour au moins chaque zone éclairée de manière à créer une matrice mono ou bidimensionnelle de valeurs de durées de vie,
- éclairer la zone de l'interface par un flux lumineux puisé provenant au moins d'un seul faisceau de lumière dont la position relative par rapport au substrat peut être changée,
- changer la position relative du faisceau de lumière pulsée par rapport à la zone de l'interface, de manière discrète ou continue,
- renouveler les étapes d'éclairage, de mesures des durées de vie, de création de la matrice et de détermination de la signature électronique, en modifiant l'un et/ou l'autre des paramètres expérimentaux suivants : l'intensité de l'éclairage, la surface de l'éclairage, la longueur d'onde de l'éclairage, la température du milieu fluide et/ou du substrat, l'intensité du champ électrique extérieure appliquée à l'interface substrat/milieu fluide, une tension appliquée entre les électrodes en contact électrique avec le milieu fluide et le substrat,
- déterminer en tant que signature électronique caractéristique du milieu fluide, au moins une image visuelle de l'interface substrat/milieu fluide, et pour au moins une valeur d'au moins un paramètre expérimental,
- découper le domaine de variation des valeurs des durées de vie enregistrées en plages à chacune desquelles une couleur est attribuée de manière que la signature électronique soit une image en couleurs,
- l'étape d'analyse consiste à comparer la signature électronique du milieu fluide à au moins une signature électronique de référence déterminée pour un milieu fluide connu,
- l'étape d'analyse consiste à utiliser des méthodes quantitatives de traitement des images obtenues,
- réaliser une étape de traitement sur la surface de réception préalablement à la réalisation de l'étape d'éclairage.

Un autre objectif de l'invention est de proposer un système pour caractériser un milieu fluide à l'aide d'un substrat semi-conducteur.

Selon l'invention, le système comporte :
- au moins une surface de réception sur le substrat pour le milieu fluide en vue de constituer une interface substrat/milieu fluide,
- un système de production d'au moins un faisceau ponctuel de lumière pulsée éclairant, à travers le milieu fluide, au moins une zone de l'interface substrat/milieu fluide pour créer des charges électriques photogénérées présentant une durée de vie dont la valeur dépend de la vitesse de recombinaison à cette interface après l'extinction de l'impulsion lumineuse,

- une source à micro-ondes,
- une antenne couplée à cette source conçue pour orienter les micro-ondes vers l'interface substrat/fluide,
- un détecteur couplé à l'antenne pour détecter les micro-ondes qui seront réfléchies par les charges électriques photogénérées,
- un système de détermination, à partir des micro-ondes réfléchies, de la durée de vie afin de créer une matrice des valeurs des durées de vie,
- un système de traitement des valeurs des durées de vie, adapté pour établir à partir de cette matrice, une signature électronique caractéristique du milieu fluide et pour analyser la signature électronique afin de reconnaître le milieu fluide et/ou l'un ou plusieurs de ses composants élémentaires.

De plus, le système selon l'invention peut comporter en outre en combinaison au moins l'une et/ou l'autre des caractéristiques additionnelles suivantes :
- un système de pilotage du faisceau ponctuel de lumière par rapport à l'interface substrat/fluide de manière à éclairer plusieurs zones de l'interface afin de mesurer des valeurs de la durée de vie des charges photogénérées pour chacune des zones de l'interface éclairée,
- le système de production du faisceau ponctuel de lumière comporte une source lumineuse ponctuelle associée à un système de déplacement relatif du faisceau par rapport à l'interface substrat/fluide.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en coupe élévation montrant le principe général de l'invention.
Les **Figures 2A** et **2B** permettent d'illustrer le mécanisme de mesure de la durée de vie de charges photoélectriques générées.
Les **Figures 3A** à **3G** donnent des exemples d'images obtenues à partir de matrices des valeurs mesurées pour l'eau en tant que milieu fluide, pour diverses tensions de polarisation.
Les **Figures 4A** à **4G** donnent des exemples d'images obtenues à partir de matrices des valeurs mesurées pour l'éthanol en tant que milieu fluide, pour diverses tensions de polarisation.

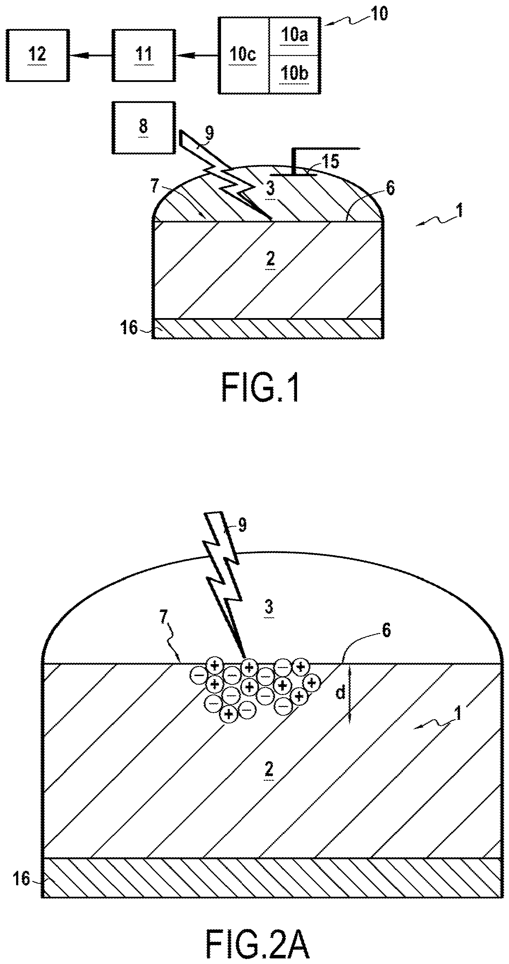

La **Fig. 1** illustre le principe d'un dispositif **1** conforme à l'invention adapté pour caractériser à l'aide d'un substrat semi-conducteur **2**, un milieu fluide **3** au sens général. Au sens de l'invention, un milieu fluide est un liquide, un gaz, un liquide gazeux, à caractère homogène ou hétérogène c'est-à-dire contenant des substances, des produits ou des composés divers comme par exemple, des objets biologiques (bactéries, cellules, virus, protéines, etc.) ou des particules à l'état solide qui peuvent être suspendus ou dissoutes dans des milieux liquides ou évaporés sous la forme d'un gaz. La caractérisation du milieu fluide s'entend ainsi de l'identification d'au moins un composé spécifique formant le milieu fluide ou contenu dans le milieu fluide. A titre d'exemples non limitatifs, il peut être utilisé en tant que milieu fluide, des produits agroalimentaires, des cosmétiques, des parfums, des produits biomédicaux, des hydrocarbures, des drogues, des substances explosives....

Le substrat semi-conducteur **2** présente une surface de réception **6** pour le milieu fluide **3** en vue de constituer une interface **7** substrat **2**/miiieu fluide **3**. Le substrat semi-conducteur **2** est réalisé par exemple, par un substrat de silicium standard de qualité microélectronique et non traité spécialement. Par exemple, la surface de réception **6** du substrat semi-conducteur **2** est naturellement oxydée dans l'air.

Le dispositif **1** selon l'invention comporte également un système de production **8** d'un faisceau ponctuel de lumière **9** pulsé et adapté pour éclairer, à travers le milieu fluide **3**, au moins une zone de l'interface **7**. Le système de production **8** est piloté pour éclairer une zone de l'interface **7**, pendant une durée déterminée. Le système de production **8** est adapté pour illuminer une ou plusieurs zones de l'interface **7** après avoir traversé le milieu fluide **3**, à l'aide d'un faisceau lumineux pulsé.

Le système de production **8** est réalisé de toute manière appropriée et peut comporter par exemple, une source lumineuse ponctuelle ou un réseau de diodes électroluminescentes pilotées en allumage/extinction.

Selon une variante de réalisation, le dispositif **1** comporte un système de pilotage du faisceau ponctuel de lumière de manière à éclairer plusieurs zones de l'interface. En particulier, le système de pilotage du faisceau ponctuel de lumière comporte un système de déplacement du système de production du faisceau ponctue! de lumière de manière à pouvoir illuminer de manière discrète ou continue des zones différentes de l'interface **7**.

Selon une autre variante de réalisation, le système de production **8** comporte un réseau de diodes électroluminescentes réparties sur une zone et qui sont pilotées sélectivement en allumage/extinction de manière à éclairer plusieurs zones différentes de l'interface **7**.

Le système **8** produit ainsi des impulsions lumineuses **9** permettant d'éclairer à travers le milieu fluide **3**, une ou plusieurs zones de l'interface **7** pour créer des charges électriques photogénérées dans le substrat semi-conducteur **2**.

Le dispositif **1** selon l'invention comporte également un réflectomètre à micro-ondes **10** pour mesurer la durée de vie des charges électriques photogénérées lors de l'illumination du substrat semi-conducteur **2**. Le réflectomètre à micro-ondes **10** est de tous types connus en soi. De manière classique, le réflectomètre **10** comporte une source à micro-ondes **10a** couplée à une antenne **10b** qui est conçue pour orienter les micro-ondes vers l'interface **7** substrat **2**/milieu fluide **3** et plus précisément au moins dans la zone de l'interface **7** éclairée par le faisceau de lumière **9**. Le réflectomètre **10** comporte également un détecteur **10c** couplé à l'antenne **10b** pour détecter les micro-ondes qui sont réfléchies par les charges électriques photogénérées lors de l'illumination du substrat semi-conducteur **2.** Le détecteur **10c** mesure ainsi la réflexion des micro-ondes induite par ces charges électriques photogénérées. Le détecteur **10C** est relié à un système de mesure **11** adapté pour déterminer, à partir de la réflexion des micro-ondes, la durée de vie de ces charges électriques et créer une matrice des durées de vie mesurées.

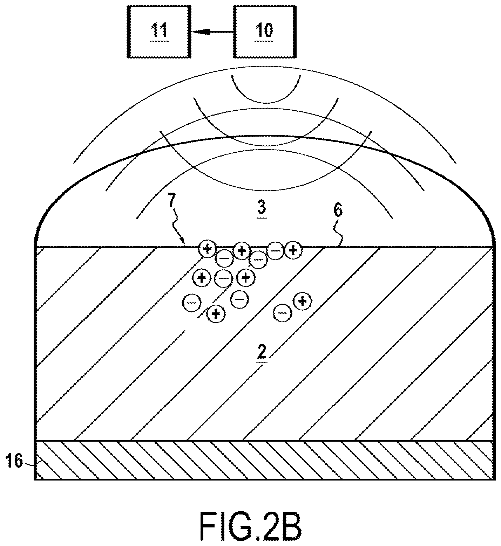

Les **Fig. 2A** et **2B** illustrent le mécanisme de mesure de la durée de vie des charges électriques photogénérées.

Comme illustré à la **Fig. 2A**, un milieu fluide **3** est mis en contact avec la surface **6** du substrat semi-conducteur **2** afin de réaliser l'interface **7** substrat/milieu fluide. L'éclairage à travers le milieu fluide **3** d'une zone de l'interface **7** pendant un temps déterminé conduit à la création à l'intérieur du substrat semi-conducteur **2** et à partir de l'interface **7**, de charges électriques (électrons e⁻ et trous e⁺). Il est à noter que la profondeur de génération d de charges électriques à l'intérieur du substrat semi-conducteur **2** dépend en particulier de la longueur d'onde du faisceau de lumière **9** et de la nature physico-chimique du substrat semi-conducteur **2**.

Après l'apparition des charges électriques à l'intérieur du substrat semi-conducteur **2**, il se produit inévitablement comme illustré à la **Fig. 2B****,** une recombinaison d'une partie des charges électriques photogénérées. Cette recombinaison est fonction de la qualité et de la nature électronique de la surface de réception **6**. Cette recombinaison d'une partie des charges électriques photogénérées est caractérisée par sa vitesse de recombinaison. Il doit être compris que les charges électriques photogénérées présentent ainsi une durée de vie entre leur apparition et leur recombinaison. Ainsi, la variation de la concentration des charges électriques photogénérées, au cours du temps due à la recombinaison à l'interface **7** dépend de leur durée de vie.

Le réflectomètre à micro-ondes **10** est utilisé pour mesurer la durée de vie des charges électriques photogénérées lors de l'éclairage de l'interface **7** à travers le milieu fluide **3**. A cet effet, un rayonnement micro-ondes dans le domaine spectral de radiofréquences est envoyé sur l'interface **7** de sorte qu'une partie de ce rayonnement se trouve réfléchie par les charges électriques photogénérées. Par exemple, l'envoi du rayonnement micro-ondes sur l'interface (**Fig. 2B**) est réalisé après l'extinction de l'impulsion lumineuse **9**. La récupération du rayonnement micro-ondes réfléchi permet de déterminer la durée de vie des charges électriques photogénérées.

Le système **11** mesure ainsi lors de chaque éclairage d'une zone de l'interface **7** et à l'aide du réflectomètre **10,** la valeur de la durée de vie des charges photoélectriques photogénérées.

Il ressort du principe décrit ci-dessus que la valeur mesurée de la durée de vie à la suite de l'éclairage de l'interface **7**, est définie directement par la vitesse de recombinaison des charges électriques photogénérées qui dépend des caractéristiques de la zone de l'interface **7** éclairée. Il peut être obtenu une sensibilité de mesure élevée avec un large domaine de valeurs pour les durées de vie mesurées dans la mesure où la vitesse de recombinaison peut varier de plusieurs ordres de grandeurs (par exemple, de 10² à 10⁶ cm/s).

La résolution spatiale de mesure, qui est responsable de la qualité de la reconnaissance ou de la caractérisation du milieu fluide et définissant la valeur minimale de la surface de réception **6**, dépend de la taille du spot lumineux **9** c'est-à-dire de la surface de la zone éclairée de l'interface **7**. La résolution spatiale de mesure dépend également du déplacement minimal de ce spot lumineux pour lequel une variation de durée de vie est détectée. En d'autres termes, la résolution spatiale dépend du degré d'hétérogénéité de la vitesse de recombinaison à la surface de réception **6**.

Selon un aspect de l'invention, il apparaît avantageux de caractériser un milieu fluide en éclairant plusieurs zones différentes de l'interface **7** qui présente naturellement ou à la suite d'un traitement spécifique, des durées de vie différentes. En effet, il a été constaté que l'interface **7** présente naturellement des vitesses de recombinaison différentes se répartissant sur la surface de cette interface **7**. Bien entendu, il peut être envisagé un traitement, par exemple, chimique de la surface de réception **6** pour obtenir des vitesses de recombinaison différentes, distribuées sélectivement sur la surface de réception **6**. Par exemple, comme traitement chimique de la surface de réception du substrat, il peut être envisagé les traitements suivants :
- oxydation, nitridation, carbonisation, porosification du substrat initial,
- dépôt des couches d'oxydes, de nitrides, de carbides ou diverses couches poreuses à la surface du substrat,
- greffages chimiques des groupements fonctionnels organiques par hydrosilylation (par exemple, surface fraiche de Si),
- greffages chimiques des groupements fonctionnels organiques par silanisation (par exemple, surface oxydée ou nitridée de Si),
- greffages électrochimiques des groupements fonctionnels organiques,
- greffage des groupements fonctionnels organiques avec des alcooles ou amines,
- reconstruction consécutives de la surface du substrat avec des groupements fonctionnels organiques,
- remplissage des pores des couches poreuses par polymères, nanoparticules (en carbones, semi-conducteurs ou métaux), liquides faiblement polaires insolubles dans l'eau,
- croissances à l'intérieur des pores ou à la surface **6** du substrat **2** des couches ou membranes sélectivement sensibles aux ions et des solutions ionophores dans les polymères.

La présence de vitesses de recombinaison différentes à l'interface **7** permet de créer une matrice des valeurs de durée de vie.

Selon une variante préférée de réalisation, le système de mesure **11** enregistre également les valeurs mesurées de la durée de vie afin de créer une matrice de valeurs correspondant aux valeurs de la durée de vie, mesurées pour les différentes zones illuminées de l'interface **7**. Le système de mesure **11** comporte à cet effet, des moyens d'enregistrement de tous types connus en soi.

La dimension de cette matrice de valeurs dépend du nombre de zones illuminées et/ou du nombre des paramètres extérieurs définis et contrôlés lors de la réalisation de ces mesures, par exemple, le temps, la température, les conditions d'éclairage, le champ électrique, etc. Par exemple, dans le cas de l'éclairage d'une seule zone et en maintenant constant les paramètres extérieurs de mesure, la matrice comporte une seule valeur, Dans le cas de l'éclairage de n zones de l'interface **7** réparties selon une direction de l'interface **7** et en maintenant constants les paramètres extérieures de mesure, il est obtenu une matrice monodimensionnelle. Dans le cas de l'éclairage de n zones de l'interface **7** réparties en lignes et en colonnes (avec n supérieur à 1) et en maintenant constants les paramètres extérieurs de mesure, la matrice des n valeurs est de dimension 2.

Le dispositif **1** selon l'invention comporte également un système **12** de traitement des valeurs de la durée de vie délivrées par le système de mesure **11**. Ce système de traitement **12** est adapté pour déterminer, à partir de la matrice des valeurs de durée de vie, une empreinte ou une signature électronique caractéristique du milieu fluide. En d'autres termes, le système de traitement **12** permet de caractériser le milieu fluide à partir des valeurs des durées de vie mesurées.

Le système de traitement **12** met en oeuvre diverses méthodes pour caractériser le milieu fluide à partir des valeurs des durées de vie mesurées. Une méthode simplifiée consiste à caractériser le milieu fluide directement à partir des valeurs mesurées de la durée de vie. Selon une variante avantageuse de réalisation, les valeurs mesurées de la durée de vie sont représentées sous la forme d'une image. Selon cette variante, la matrice des valeurs de la durée de vie est convertie ou représentée sous la forme d'une image comportant une ou plusieurs couleurs avec éventuellement des nuances dans les couleurs. Ainsi, le domaine de variation des valeurs de la durée de vie est découpée en plages à chacune desquelles une couleur ou nuance de couleur est attribuée de sorte qu'à partir d'une matrice de valeurs à deux dimensions est obtenue une image en couleurs correspondante également à deux dimensions.

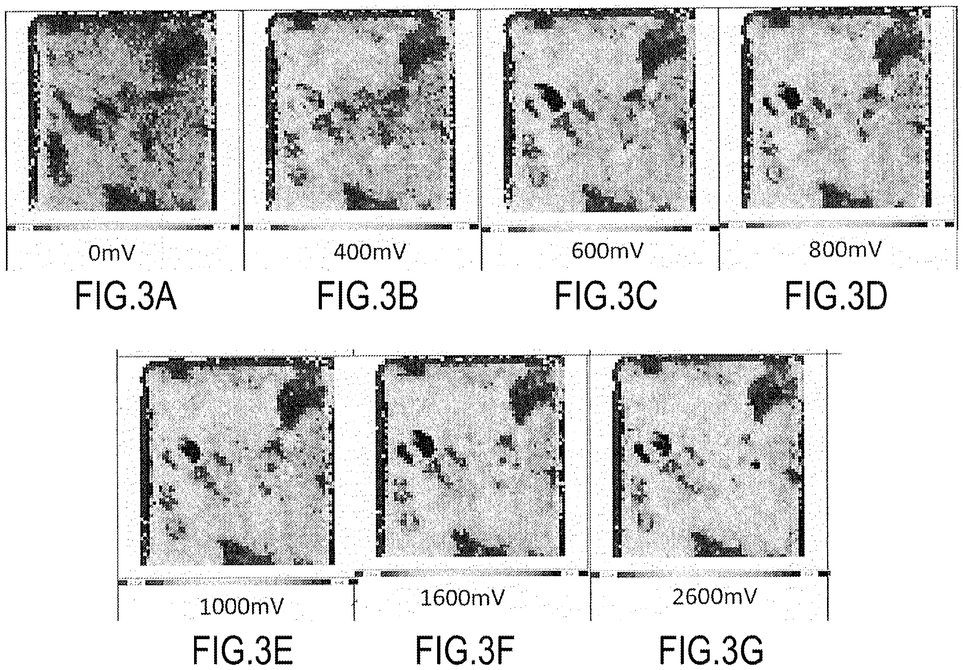

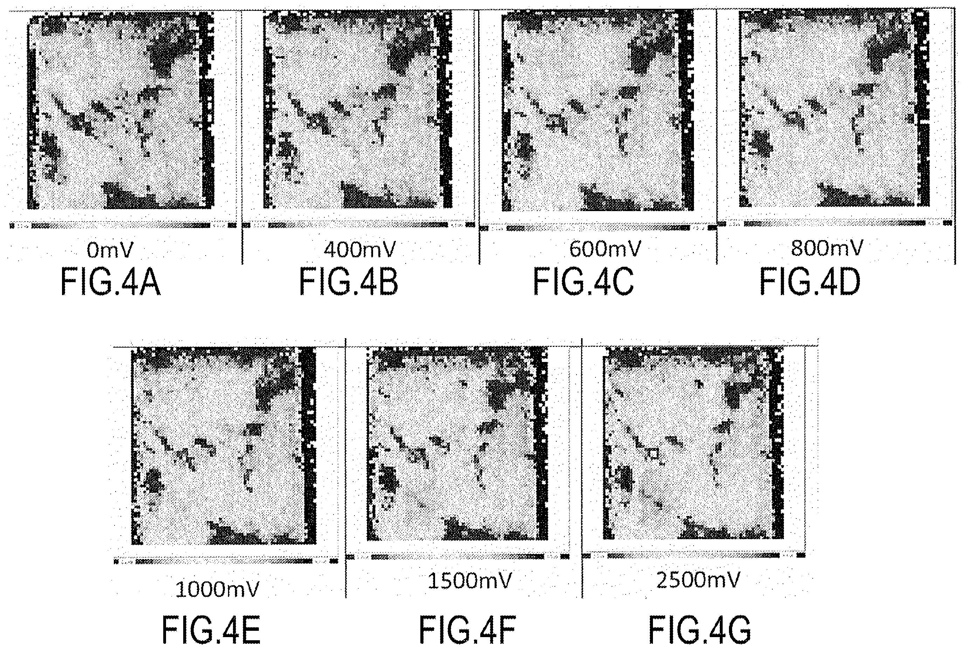

Les **Fig. 3A** et **4A** sont des exemples d'images obtenues à partir d'une matrice des valeurs de la durée de vie des charges électriques photogénérées, mesurées respectivement pour l'eau et l'éthanol en tant que milieu fluide pour une valeur de la tension de polarisation égale à 0 mV. Les zones les plus claires des images correspondent aux plus grandes valeurs de la matrice.

La suite de la description donnera d'autres exemples permettant d'illustrer l'objet de l'invention qui est mis en oeuvre essentiellement selon plusieurs variantes de réalisation.

Selon une première variante de réalisation, le substrat semi-conducteur **2** comporte une première électrode **15** plongée dans le milieu fluide **3** et une deuxième électrode **16** réalisée sur la face du substrat semi-conducteur opposée de celle formant l'interface **7**. Les électrodes **15**, **16** sont reliées à une source d'alimentation électrique permettant d'appliquer une tension de polarisation sur l'ensemble milieu fluide **3** et substrat semi-conducteur **2**.

Avantageusement, l'objet de l'invention vise à créer des matrices de durée de vie et par suite des signatures électroniques pour différentes valeurs de la tension appliquée entre les électrodes **15**, **16**.

Les **Fig. 3A** à **3G** illustrent un exemple d'images obtenues à partir de la durée de vie des charges électriques photogénérées mesurées pour l'eau pour respectivement les valeurs de tension de polarisation : 0, 400, 600, 800, 1 000, 1 600, et 2 600 mV.

Les **Fig. 4A** à **4G** illustrent un exemple d'images obtenues à partir de la durée de vie des charges électriques photogénérées mesurées pour l'éthanol pour respectivement les valeurs de tension de polarisation : 0, 400, 600, 800, 1 000, 1 600, et 2 500 mV.

Il ressort de la description qui précède que le dispositif **1** selon l'invention permet de caractériser ou d'identifier un milieu fluide à partir des valeurs de la durée de vie des charges électriques qui sont caractéristiques dudit milieu fluide. La méthode pour identifier un milieu fluide découle directement de la description qui précède.

Le procédé selon l'invention consiste ainsi :
- à réaliser une surface de réception **6** sur le substrat **2** pour le milieu fluide **3** sur au moins une face du substrat,
- à mettre le milieu fluide **3** à caractériser en contact avec la surface de réception, afin de réaliser une interface **7** substrat/milieu fluide,
- à éclairer à travers le milieu fluide **3**, au moins une zone de l'interface à l'aide d'un faisceau de lumière pulsé pour créer des charges électriques photogénérées,
- en utilisant un réflectomètre à micro-ondes **10**, mesurer la durée de vie des charges électriques photogénérées dont la valeur dépend de la vitesse de recombinaison à l'interface **7** substrat/milieu fluide,
- à créer une matrice des valeurs des durées de vie mesurées, des charges électriques photogénérées,
- à partir de la matrice des durées de vie créée, déterminer une signature électronique caractéristique du milieu fluide,
- et à analyser cette signature électronique afin de reconnaître le milieu fluide **3** et/ou l'un ou plusieurs de ses composants élémentaires.

Il doit être compris que le procédé selon l'invention permet d'identifier un milieu fluide à partir de la matrice des valeurs de la durée de vie des charges électriques photogénérées, formant une signature électronique unique pour chaque milieu fluide. Bien entendu, pour augmenter la qualité de détection, le procédé de détection consiste à renouveler les étapes d'éclairage, de récupération de la valeur de durée de vie, de création de la matrice et de détermination de la signature électronique, soit en maintenant les divers paramètres expérimentaux soit en modifiant l'un et/ou l'autre des paramètres expérimentaux suivants : l'intensité de l'éclairage, la surface de l'éclairage, la longueur d'onde de l'éclairage, la température du milieu fluide et/ou du substrat, l'intensité du champ électrique extérieure appliqué à l'interface substrat/milieu fluide, une tension appliquée entre des électrodes en contact électrique avec le substrat **2** et le milieu fluide **3**.

De préférence, cette signature électronique est convertie en une image visuelle. L'identification du milieu fluide à partir d'une ou de plusieurs images peut être réalisée à l'oeil nu, par comparaison à une signature électronique de référence déterminée pour un milieu fluide connu ou à l'aide de divers traitements d'images et en particulier en utilisant des méthodes quantitatives de traitement des images obtenus. La signature électronique est analysée afin de reconnaître le milieu fluide **3** et/ou l'un ou plusieurs de ses composants élémentaires.

## Revendications

1. Procédé pour caractériser un milieu fluide (**3**) comportant les étapes suivantes :
- réaliser une surface de réception (**6**) pour le milieu fluide (**3**) sur au moins une face d'un substrat semi-conducteur (**2**),
- mettre le milieu fluide en contact avec la surface de réception (**6**), afin de réaliser une interface (**7**) substrat/milieu fluide,
- éclairer à travers le milieu fluide (**3**), au moins une zone de l'interface (**7**) à l'aide d'un faisceau de lumière puisé (**9**) pour créer des charges électriques photogénérées,
- en utilisant un réflectomètre à micro-ondes (**10**), mesurer la durée de vie des charges électriques photogénérées dont la valeur dépend de la vitesse de recombinaison à l'interface (**7**) substrat/milieu fluide,
- créer une matrice des valeurs des durées de vie mesurées, des charges électriques photogénérées,
- déterminer une signature électronique caractéristique du milieu fluide à partir de la matrice des durées de vie créée, **caractérisé en ce qu'**il consiste pour caractériser le milieu fluide (3),
- à analyser la signature électronique afin de reconnaitre le milieu fluide (**3**) et/ou l'un ou plusieurs de ses composants élémentaires.

2. Procédé selon la revendication 1, **caractérisé en ce qu'**il consiste à éclairer successivement dans le temps, plusieurs zones de l'interface (**7**) et à déterminer les durées de vie correspondantes des charges électriques photogénérées, pour au moins chaque zone éclairée de manière à créer une matrice mono ou bidimensionnelle de valeurs de durées de vie.

3. Procédé selon la revendication 1, **caractérisé en ce qu'**il consiste à éclairer la zone de l'interface (**7**) par un flux lumineux pulsé (**9**) provenant au moins d'un seul faisceau de lumière dont la position relative par rapport au substrat peut être changée.

4. Procédé selon l'une des revendications 1 à 3, **caractérisé en ce qu'**il consiste à changer la position relative du faisceau de lumière pulsée (**9**) par rapport à la zone de l'interface (**7**), de manière discrète ou continue.

5. Procédé selon l'une des revendications 1 à 4, **caractérisé en ce qu'**il consiste à renouveler les étapes d'éclairage, de mesures des durées de vie, de création de la matrice et de détermination de la signature électronique, en modifiant l'un et/ou l'autre des paramètres expérimentaux suivants : l'intensité de l'éclairage, la surface de l'éclairage, la longueur d'onde de l'éclairage, la température du milieu fluide et/ou du substrat, l'intensité du champ électrique extérieure appliquée à l'interface (**7**) substrat/milieu fluide, une tension appliquée entre les électrodes (**15**, **16**) en contact électrique avec le milieu fluide (**3**) et le substrat (**2**).

6. Procédé selon la revendication 5, **caractérisé en ce qu'**il consiste à déterminer en tant que signature électronique caractéristique du milieu fluide, au moins une image visuelle de l'interface substrat/milieu fluide, et pour au moins une valeur d'au moins un paramètre expérimental

7. Procédé selon l'une des revendications 1 à 6, **caractérisé en ce qu'**il consiste à découper le domaine de variation des valeurs des durées de vie enregistrées en plages à chacune desquelles une couleur est attribuée de manière que la signature électronique soit une image en couleurs.

8. Procédé selon la revendication 1, **caractérisé en ce que** l'étape d'analyse consiste à comparer la signature électronique du milieu fluide (**3**) à au moins une signature électronique de référence déterminée pour un milieu fluide connu.

9. Procédé selon la revendication 1, **caractérisé en ce que** l'étape d'analyse consiste à utiliser des méthodes quantitatives de traitement des images obtenues.

10. Procédé selon l'une des revendications précédentes **caractérisé en ce qu'**il consiste à réaliser une étape de traitement sur la surface de réception (**6**) préalablement à la réalisation de l'étape d'éclairage.

11. Système pour caractériser un milieu fluide (**3**) à l'aide d'un substrat semi-conducteur (**2**) **caractérisé en ce qu'**il comporte :
- au moins une surface de réception (**6**) sur le substrat (**2**) pour le milieu fluide (**3**) en vue de constituer une interface (**7**) substrat/milieu fluide,
- un système de production (**8**) d'au moins un faisceau ponctuel (**9**) de lumière puisée éclairant, à travers le milieu fluide (**3**), au moins une zone de l'interface (**7**) substrat/milieu fluide pour créer des charges électriques photogénérées présentant une durée de vie dont la valeur dépend de la vitesse de recombinaison à cette interface après l'extinction de l'impulsion lumineuse,
- une source à micro-ondes (**10a**),
- une antenne (**10b**) couplée à cette source conçue pour orienter les micro-ondes vers l'interface substrat/fluide,
- un détecteur (**10c**) couplé à l'antenne pour détecter les micro-ondes qui seront réfléchies par les charges électriques photogénérées,
- un système de détermination (**11**), à partir des micro-ondes réfléchies, de la durée de vie afin de créer une matrice des valeurs des durées de vie,
- un système de traitement (**12**) des valeurs des durées de vie, adapté pour établir à partir de cette matrice, une signature électronique caractéristique du milieu fluide, **caractérisé en ce que** le système de traitement (**12**) est adapté pour analyser la signature électronique afin de reconnaitre le milieu fluide (**3**) et/ou l'un ou plusieurs de ses composants élémentaires.

12. Système selon la revendication 11, **caractérisé en ce qu'**il comporte un système de pilotage du faisceau ponctuel de lumière (**9**) par rapport à l'interface (**7**) substrat/fluide de manière à éclairer plusieurs zones de l'interface afin de mesurer des valeurs de la durée de vie des charges photogénérées pour chacune des zones de l'interface éclairée.

13. Système selon les revendications 11 et 12, **caractérisé en ce que** le système de production (**8**) du faisceau ponctuel de lumière (**9**) comporte une source lumineuse ponctuelle associée à un système de déplacement relatif du faisceau par rapport à l'interface substrat/fluide.

## Patentansprüche

1. Verfahren zur Charakterisierung eines flüssigen Mediums (3), das die folgenden Schritte umfasst:
- eine Aufnahmefläche (6) für das flüssige Medium (3) auf zumindest einer Seite eines Halbleitersubstrats (2) schaffen,
- das flüssige Medium mit der Aufnahmefläche (6) in Berührung bringen, um eine Schnittstelle (7) zwischen Substrat und flüssigem Medium zu schaffen,
- durch das flüssige Medium (3) hindurch zumindest einen Bereich der Schnittstelle (7) mit einem gepulsten Lichtstrahl (9) beleuchten, um durch Lichteinwirkung erzeugte elektrische Ladungen zu schaffen,
- unter Verwendung eines Mikrowellenreflektometers (10) die Lebensdauer der durch Lichteinwirkung erzeugten elektrischen Ladungen messen, deren Wert von der Rekombinationsrate an der Schnittstelle (7) Substrat/flüssiges Medium abhängt,
- eine Matrix der gemessenen Lebensdauerwerte der durch Lichteinwirkung erzeugten elektrischen Ladungen erstellen,
- aus der erzeugten Lebensdauer-Matrix eine charakteristische elektronische Signatur des flüssigen Mediums bestimmen,
**dadurch gekennzeichnet, dass** es darin besteht, das flüssige Medium (3) zu charakterisieren,
- die elektronische Signatur zu analysieren, um das flüssige Medium (3) und/oder eine oder mehrere seiner elementaren Komponenten zu erkennen.

2. Verfahren nach Anspruch 1, **dadurch gekennzeichnet, dass** es darin besteht, mehrere Bereiche der Schnittstelle (7) zeitlich nacheinander zu beleuchten und die entsprechenden Lebensdauern der durch Lichteinwirkung erzeugten elektrischen Ladungen für zumindest jede beleuchtete Fläche so zu bestimmen, dass eine ein- oder zweidimensionale Matrix von Lebensdauerwerten entsteht.

3. Verfahren nach Anspruch 1, **dadurch gekennzeichnet, dass** es darin besteht, den Schnittflächenbereich (7) mit einem gepulsten Lichtstrom (9) zu beleuchten, der von zumindest einem einzigen Lichtstrahl kommt, dessen relative Position zum Substrat veränderbar ist.

4. Verfahren nach einem der Ansprüche 1 bis 3, **dadurch gekennzeichnet, dass** es darin besteht, die relative Position des gepulsten Lichtstrahls (9) in Bezug auf den Schnittstellenbereich (7) diskret oder kontinuierlich zu verändern.

5. Verfahren nach einem der Ansprüche 1 bis 4, **dadurch gekennzeichnet, dass** es darin besteht, die Schritte der Beleuchtung, der Lebensdauermessung, der Matrixerstellung und der elektronischen Signaturbestimmung zu erneuern, indem der eine und/oder der andere der folgenden Versuchsparameter geändert wird: die Intensität der Beleuchtung, die Beleuchtungsfläche, die Wellenlänge der Beleuchtung, die Temperatur des flüssigen Mediums und/oder Substrats, die Intensität des äußeren elektrischen Feldes, das an die Schnittstelle (7) zwischen dem Substrat und dem flüssigen Medium angelegt wird, eine Spannung, die zwischen den Elektroden (15, 16) in elektrischem Kontakt mit dem flüssigen Medium (3) und dem Substrat (2) angelegt wird.

6. Verfahren nach Anspruch 5, **dadurch gekennzeichnet, dass** es darin besteht, als charakteristische elektronische Signatur des flüssigen Mediums zumindest ein visuelles Bild der Schnittstelle Substrat/Flüssigmedium, und für zumindest einen Wert zumindest eines experimentellen Parameters zu bestimmen.

7. Verfahren nach einem der Ansprüche 1 bis 6, **dadurch gekennzeichnet, dass** es darin besteht, den Variationsbereich der aufgezeichneten Lebensdauerwerte in Bereiche zu schneiden, denen jeweils eine Farbe zugeordnet ist, so dass die elektronische Signatur ein Farbbild ist.

8. Verfahren nach Anspruch 1, **dadurch gekennzeichnet, dass** der Analyseschritt darin besteht, die elektronische Signatur des flüssigen Mediums (3) mit zumindest einer bestimmten elektronischen Referenzsignatur für ein bekanntes flüssiges Medium zu vergleichen.

9. Verfahren nach Anspruch 1, **dadurch gekennzeichnet, dass** der Analyseschritt darin besteht, quantitative Verfahren für die Verarbeitung der erzielten Bilder zu benutzen.

10. Verfahren nach einem der vorstehenden Ansprüche, **dadurch gekennzeichnet, dass** es darin besteht, vor der Ausführung des Schritts der Beleuchtung einen Schritt der Verarbeitung auf der Aufnahmefläche (6) durchzuführen.

11. System zur Charakterisierung eines flüssigen Mediums (3) mit Hilfe eines Halbleitersubstrats (2), **dadurch gekennzeichnet, dass** es Folgendes umfasst:
- zumindest eine Aufnahmefläche (6) auf dem Substrat (2) für das flüssige Medium (3), um eine Schnittstelle (7) Substrat/flüssiges Medium zu bilden,
- ein System (8) zum Erzeugen zumindest eines punktuellen gepulsten Lichtstrahls (9), der durch das flüssige Medium (3) hindurch zumindest eine Zone der Schnittstelle (7) Substrat/flüssiges Medium beleuchtet, um durch Lichteinwirkung erzeugte elektrische Ladungen zu erzeugen, die eine Lebensdauer aufweisen, deren Wert von der Rekombinationsrate an dieser Schnittstelle nach dem Verlöschen des Lichtimpulses abhängt,
- eine Mikrowellenquelle (10a),
- eine mit dieser Quelle gekoppelte Antenne (10b), die dazu konzipiert ist, die Mikrowellen auf die Schnittstelle Substrat/Flüssigkeit zu richten,
- einen mit der Antenne gekoppelten Detektor (10c), um die Mikrowellen zu erfassen, die durch die mit Lichteinwirkung erzeugten elektrischen Ladungen reflektiert werden,
- ein System zur Bestimmung (11) der Lebensdauer aus den reflektierten Mikrowellen, um eine Matrix der Lebensdauerwerte zu erzeugen,
- ein System (12) zur Verarbeitung von Lebensdauerwerten, das geeignet ist, aus dieser Matrix eine für das flüssige Medium charakteristische elektronische Signatur zu erstellen, **dadurch gekennzeichnet, dass** das Verarbeitungssystem (12) geeignet ist, die elektronische Signatur zu analysieren, um das flüssige Medium (3) und/oder eine oder mehrere seiner elementaren Komponenten zu erkennen.

12. System nach Anspruch 11, **dadurch gekennzeichnet, dass** es ein System zur Steuerung des punktuellen gepulsten Lichtstrahls (9) in Bezug auf die Schnittstelle (7) Substrat/Flüssigkeit umfasst, um mehrere Bereiche der Schnittstelle zu beleuchten, um Werte der Lebensdauer der durch Lichteinwirkung erzeugten elektrischen Ladungen für jeden der Bereiche der beleuchteten Schnittstelle zu messen.

13. System nach den Ansprüchen 11 und 12, **dadurch gekennzeichnet, dass** das System (8) zur Erzeugung des punktuellen gepulsten Lichtstrahls (9) eine punktuelle Lichtquelle umfasst, die einem System der relativen Bewegung des Strahls gegenüber der Schnittfläche Substrat/Flüssigkeit zugeordnet ist.

## Claims

1. A method of characterizing a fluid medium (3), the method comprising the following steps:
- making a reception surface (6) for receiving the fluid medium (3) on at least one face of a semiconductor substrate (2),
- putting the fluid medium into contact with the reception surface (6) in order to make an interface (7) between the substrate and the fluid medium,
- lighting at least one zone of the interface (7) through the fluid medium (3) with the help of a pulsed light beam (9) in order to create photogenerated electric charges,
- using a microwave reflectometer (10) to measure the lifetime durations of the photogenerated electric charges, which durations have respective values that depend on the recombination rate at the interface (7) between the substrate and the fluid medium,
- creating a matrix of measured lifetime duration values for the photogenerated electric charges,
- determining an electronic signature that is characteristic of the fluid medium from the lifetime duration matrix that has been created,
the method being **characterized in that** for the purpose of characterizing the fluid medium (3) it consists in:
- analyzing the electronic signature in order to recognize the fluid medium (3) and/or one or more of its elementary components.

2. The method according to claim 1, **characterized in that** it consists in lighting a plurality of zones of the interface (7) successively over time and in determining the corresponding lifetime durations for the photogenerated electric charges for at least each lighted zone in order to create a one- or two-dimensional matrix of lifetime duration values.

3. The method according to claim 1, **characterized in that** it consists in lighting the interface zone (7) with pulsed light flux (9) coming from at least a single light beam of position relative to the substrate that can be changed.

4. The method according to one of claims 1 to 3, **characterized in that** it consists in changing the position of the pulsed light beam (9) relative to the zone of the interface (7), in a manner that is discrete or continuous.

5. The method according to one of claims 1 to 4, **characterized in that** it consists in renewing the steps of lighting, measuring lifetime durations, creating the matrix, and determining the electronic signature, while modifying one or more of the following experimental parameters: lighting intensity, lighting area, lighting wavelength, temperature of the fluid medium and/or of the substrate, intensity of the external electric field applied to the interface (7) between the substrate and the fluid medium, and a voltage applied between the electrodes (15, 16) in electrical contact with the fluid medium (3) and with the substrate (2).

6. The method according to claim 5, **characterized in that** it consists in determining as the electronic signature characteristic of the fluid medium at least one visual image of the interface between the substrate and the fluid medium, and for at least one value of at least one experimental parameter.

7. The method according to one of claims 1 to 6, **characterized in that** it consists in subdividing the field over which the measured lifetime duration values vary into areas, each of which has a color allocated thereto so that the electronic signature is a color image.

8. The method according to claim 1, **characterized in that** the step of analyzing the signature consists in comparing the electronic signature of the fluid medium (3) with at least one reference electronic signature determined for a known fluid medium.

9. The method according to claim 1, **characterized in that** the step of analyzing the signature consists in using quantitative methods for processing the images obtained.

10. The method according to one of the preceding claims, **characterized in that** it consists in performing a step of treating the reception surface (6) prior to performing the lighting step.

11. A system for characterizing a fluid medium (3) with the help of a semiconductor substrate (2), the system being **characterized in that** it comprises:
- at least one reception surface (6) on the substrate (2) for receiving the fluid medium (3) in order to constitute an interface (7) between the substrate and the fluid medium,
- a production system (8) for producing at least one pulsed spot light beam (9) for lighting at least one zone of the interface (7) between the substrate and the fluid medium through the fluid medium (3) in order to create photogenerated electric charges presenting lifetime durations of respective values that depend on rate of recombination at the interface after extinction of the light pulse,
- a microwave source (10a),
- an antenna (10b) coupled to the source and designed to direct the microwaves towards the interface between the substrate and the fluid,
- a detector (10c) coupled to the antenna in order to detect microwaves that are reflected by the photogenerated electric charges,
- a determination system (11) responsive to the reflected microwaves to determine the lifetime durations in order to create a matrix of lifetime duration values,
- a processor system (12) for processing lifetime duration values and adapted to use the matrix to establish an electronic signature characteristic of the fluid medium, **characterized in that** the processor system (12) is adapted to analyze the electronic signature in order to recognize the fluid medium (3) and/or one or more of its elementary components.

12. The system according to claim 11, **characterized in that** it includes a control system for controlling the spot light beam (9) relative to the interface (7) between the substrate and the fluid in such a manner as to light a plurality of zones of the interface so as to measure lifetime duration values for photogenerated electric charges at each of the lighted interface zones.

13. The system according to claims 11 and 12, **characterized in that** the production system (8) for producing the spot light beam (9) comprises a spot light source associated with a system for moving the beam relative to the interface between the substrate and the fluid.
